# EUROPEAN PATENT APPLICATION

(11) **EP 4 480 478 A1**
(43) Date of publication of application: **25.12.2024**
(21) Application number: 22925253.1
(22) Date of filing: 30.11.2022
(51) Int. Cl.: A61K 31/40, A61P 25/18, A61P 25/28, A61P 25/32, C12N 5/071, C12Q 1/02, G01N 33/15, G01N 33/50, G01N 33/68

(54) **SCREENING METHOD AND THERAPEUTIC AGENT FOR MENTAL DISORDER**

(30) Priority: 14.02.2022 JP 2022020298
(71) Applicant: Fujita Academy, Toyoake-shi, Aichi 470-1192 (JP)
(72) Inventor: KUNISAWA, Kazuo, Toyoake-shi Aichi 470-1192 (JP); MOURI, Akihiro, Toyoake-shi Aichi 470-1192 (JP); NABESHIMA, Toshitaka, Toyoake-shi Aichi 470-1192 (JP)
(74) Representative: Hertin und Partner Rechts- und Patentanwälte PartG mbB
(86) International application number: PCT/JP2022/044170
(87) International publication number: WO 2023/153055

(57) **Abstract**

Provided are a novel therapeutic agent for a mental disorder and a screening method for a therapeutic agent for a mental disorder. The therapeutic agent for a mental disorder includes clemastine or a pharmacologically acceptable salt thereof as an active ingredient. According to the therapeutic agent of the present invention, myelin sheath abnormality and brain dysfunction due to a mental disorder can be mitigated. Further, through use of the screening method of the present invention, a therapeutic agent for a mental disorder can be screened for.

## Description

### Technical Field

The present invention relates to a therapeutic agent and a screening method for a mental disorder.

The present application claims priority from Japanese Patent Application No. 2022-020298, which is incorporated herein by reference.

### Background Art

Clemastine, which is known as a first-generation antihistamine drug, has been also reported as a drug for enhancing oligodendrocyte differentiation (Non Patent Literature 1). Moreover, it has been reported that administration of clemastine to patients with multiple sclerosis (MS), which is a typical chronic demyelinating disease, proved significant safety and efficacy of clemastine, while the possibility that clemastine may stimulate the differentiation from oligodendrocyte progenitor cells to mature oligodendrocytes has been suggested by in vitro, animal model, and human cell studies (Non Patent Literatures 1 and 2) . Further, in Patent Literatures 1 and 2, there are disclosures of methods of differentiating oligodendrocytes with clemastine or the like. However, the relationship between a mental disorder and oligodendrocytes has not been known well.

Oligodendrocytes are one type of glial cells in the central nervous system and responsible for myelin sheath formation. The oligodendrocytes have a main function of inducing saltatory conduction by the myelin sheath formation, to thereby increase the conduction speed of an action potential. When the oligodendrocytes are damaged, nerve conduction is reduced, which causes abnormality in the brain function.

The number of patients requiring treatment for an alcohol use disorder, which is a mental disorder, is estimated to be extremely large, specifically, about one million. The alcohol use disorder due to chronic alcohol intake is associated with problems, such as irreversible brain dysfunctions (such as cognitive and memory disorders, and an anxiety disorder). The alcohol use disorder, for which an effective therapeutic method has not been established yet, has been treated yet with symptomatic therapies, such as an alcohol deterrent and counselling. Meanwhile, for the treatment of schizophrenia, which is a mental disorder, an anti-schizophrenia drug has been used, but the current drug therapy is mainly for mitigating symptoms. Accordingly, there has been a call for development of a novel therapeutic agent for a mental disorder with less or no side effects.

### Citation List

### Patent Literature

[PTL 1] WO 2012/112933 A1
[PTL 2] WO 2019/195742 A1

### Non Patent Literature

[NPL 1] Mei F et al., Nat Med., Aug, 2014 20(8): 954-960
[NPL 2] Green AJ et al., Lancet, December 2, 2017; 390: 2481-2489

### Summary of Invention

### Technical Problem

The present invention has an object of providing a novel therapeutic agent for a mental disorder and a screening method for a therapeutic agent for a mental disorder.

### Solution to Problem

The inventors of the present invention have made extensive investigations to achieve the above-mentioned object, and as a result, have found that the object is achieved by a therapeutic agent for a mental disorder, which includes clemastine or a pharmacologically acceptable salt thereof as an active ingredient. Further, the inventors have, for the first time, found abnormal differentiation of oligodendrocytes and myelin sheath abnormality in an alcohol use disorder. Based on those findings, the inventors have completed the present invention.

That is, the present invention includes the following.
1. A therapeutic agent for a mental disorder, including clemastine or a pharmacologically acceptable salt thereof as an active ingredient.
2. The therapeutic agent according to the above-mentioned item 1, wherein the mental disorder is an alcohol use disorder and/or schizophrenia.
3. The therapeutic agent according to the above-mentioned item 2, wherein the alcohol use disorder is alcoholic brain dysfunction and/or myelin sheath abnormality.
4. A screening method for a therapeutic agent for a mental disorder, including screening for an oligodendrocyte differentiation enhancing substance.
5. The screening method for a therapeutic agent for a mental disorder according to the above-mentioned item 4, including a step of measuring an expression amount of an oligodendrocyte marker.
6. The screening method according to the above-mentioned item 4 or 5, wherein the mental disorder is an alcohol use disorder and/or schizophrenia.
7. The screening method according to the above-mentioned item 6, wherein the alcohol use disorder is alcoholic brain dysfunction and/or myelin sheath abnormality.
8. The screening method according to the above-mentioned item 4 or 5, further including a step of collecting a test sample from a subject to which a candidate substance has been administered, or a step of incubating oligodendrocytes with a candidate substance added thereto.
9. The screening method according to the above-mentioned item 4 or 5, including at least the following steps (1) to (3):
   (1) collecting a test sample from a subject to which a candidate substance has been administered;
   (2) measuring, in the test sample, an expression amount of each of one or a plurality of kinds of oligodendrocyte markers selected from MAG, CNPase, CC1, Olig2, MBP, PDGFRα, and NG2; and
   (3) selecting the candidate substance as the oligodendrocyte differentiation enhancing substance when the expression amount of each of the one or the plurality of oligodendrocyte markers is enhanced in the subject with the candidate substance administered thereto as compared to a subject without the candidate substance.
10. A method of examining prognosis of a mental disorder, including measuring, in a test sample collected from a human subject, one or a plurality of kinds selected from oligodendrocyte markers, oligodendrocyte differentiation modulators, oligodendrocyte differentiation regulators, and oligodendrocyte-derived products.

A. A method of treating a mental disorder, including administering an effective amount of clemastine or a pharmacologically acceptable salt thereof.
B. The treatment method according to the above-mentioned item A, wherein the mental disorder is an alcohol use disorder and/or schizophrenia.
C. The treatment method according to the above-mentioned item B, wherein the alcohol use disorder is an alcoholic brain dysfunction and/or myelin sheath abnormality.

### Advantageous Effects of Invention

According to the therapeutic agent for a mental disorder of the present invention, the therapeutic agent including clemastine or a pharmacologically acceptable salt thereof as an active ingredient, the myelin sheath abnormality and/or brain dysfunction due to a mental disorder can be mitigated. Further, through use of the screening method of the present invention, the therapeutic agent for a mental disorder can be screened for.

### Brief Description of Drawings

FIG. 1(a) is an illustration of an administration schedule of intermittent alcohol intake of mice and a behavior evaluation schedule immediately after the alcohol intake. FIG. 1(b) shows behavior evaluation of the mice immediately after the alcohol intake. (Example 1)
FIG. 2(a) is an illustration of an administration schedule of intermittent alcohol intake of mice and a behavior evaluation schedule after alcohol intake and alcohol abstinence. FIG. 2(b) shows behavior evaluation of the mice after the alcohol intake and alcohol abstinence. (Example 2)
FIG. 3 shows myelin sheath abnormalities in mice immediately after the alcohol intake and after the alcohol intake and alcohol abstinence. (Example 3)
FIG. 4(a) shows the numbers of oligodendrocyte progenitor cells and mature oligodendrocytes counted in the mice immediately after the alcohol intake. (Example 4)
FIG. 4(b) shows the numbers of oligodendrocyte progenitor cells and mature oligodendrocytes counted in the mice after the alcohol intake and alcohol abstinence. (Example 4)
FIG. 5 shows the effect of clemastine on the mice after the alcohol intake and alcohol abstinence. (Example 5)
FIG. 6(a) is an illustration of an administration schedule of intermittent alcohol intake of mice. FIG. 6(b) shows an abnormality in differential proliferation of oligodendrocytes in the mice after alcohol intake and alcohol abstinence. FIG. 6(c) shows the numbers of co-positive oligodendrocytes for an oligodendrocyte marker (Olig2) and a differential proliferation marker (BrdU). (Example 6)
FIG. 7(a) is an illustration of an administration schedule of intermittent alcohol intake of NG2-CreERT;Myrf-Flox mice, and behavior evaluation schedule of the mice after the alcohol intake and alcohol abstinence. FIG. 7(b) shows behavior evaluation of the NG2-CreERT;Myrf-Flox mice after the alcohol intake and alcohol abstinence. (Example 7)
FIG. 8(a) is an illustration of an administration schedule of clemastine to mice after intermittent alcohol intake, and remission of myelin sheath abnormality in the mice after alcohol intake and alcohol abstinence. FIG. 8(b) and FIG. 8(c) show the effect of clemastine on the myelin sheath abnormality after the alcohol intake and alcohol abstinence. (Example 8)

### Description of Embodiments

The present invention relates to a therapeutic agent for a mental disorder, including clemastine or a pharmacologically acceptable salt thereof as an active ingredient.

The therapeutic agent of the present invention includes clemastine, which is known as an antihistamine drug, or a pharmacologically acceptable salt thereof as an active ingredient. Examples of the "pharmacologically acceptable salt thereof" as used herein include a salt with an inorganic acid, a salt with an organic acid, and a salt with a basic or acidic amino acid. Examples of the salt with an inorganic acid include salts with hydrochloric acid, hydrobromic acid, nitric acid, sulfuric acid, and phosphoric acid. Examples of the salt with an organic acid include salts with fumaric acid, acetic acid, trifluoroacetic acid, oxalic acid, tartaric acid, maleic acid, citric acid, succinic acid, malic acid, methanesulfonic acid, benzoic acid, and toluenesulfonic acid salts. In particular, a salt with fumaric acid is preferred.

The term "mental disorder" as used herein refers to a state having a functional disorder with a specific mental or behavioral symptom, and refers to those listed in Diagnostic and Statistical Manual-5 (DSM-5). Examples of such state include states presenting various symptoms including a substance-related disorder such as drug dependence such as an alcohol use disorder, schizophrenia, depression, a bipolar disorder, an anxiety disorder such as a panic disorder, and sexual dysfunction. Accordingly, examples of the "mental disorder" include an alcohol use disorder, schizophrenia, depression, manic depression, a bipolar disorder, an anxiety disorder, an impulse disorder, bulimia, a panic disorder, a social anxiety disorder, insomnia, attention deficit hyperactivity disorder (ADHD), dementia, a personality disorder, a dissociative disorder, sleep apnea syndrome, and fibromyalgia. In particular, an alcohol use disorder and/or schizophrenia is preferred.

The term "alcohol use disorder" as used herein refers to a state in which some mental or physical disorder occurs with alcohol intake. It is particularly preferred that alcoholic brain dysfunction and/or myelin sheath abnormality occur. Examples of the "alcohol use disorder" as used herein include alcoholism, a state which has not been yet aggregated to alcoholism but in which some mental or physical disorder has occurred due to alcohol intake (harmful alcohol use), a state in which a social or familial problem has occurred due to alcohol intake (alcohol abuse), and a state in which some alcohol-related problem has occurred even without experience of withdrawal symptoms or continuous alcohol intake (pre-alcoholism). The states in which some mental or physical disorder has occurred due to alcohol intake may be a state during alcohol intake or under alcohol abstinence. The term "alcohol abstinence" as used herein refers to a state in which further alcohol intake is stopped after alcohol intake. The term "alcohol" as used herein refers to a main ingredient of an alcoholic drink such as so-called "sake" generally in Japanese, is a collective term for derivatives of hydrocarbons with a hydrogen atom replaced by a -OH group, and specifically means ethanol (EtOH). Examples of the "mental disorder" as used herein include brain dysfunction, myelin sheath abnormality, auditory hallucination, and a sleep disorder. Examples of the "physical disorder" include high blood pressure, arrhythmia, and alcoholic liver disease.

A method of diagnosing the alcohol use disorder is, for example, DSM-5. According to the DSM-5, when a patient has experienced two or more of the following states within 12 months, the patient is diagnosed as an alcohol use disorder.
·The patient uses alcohol in a larger amount or for a longer period than intended.
·The patient tries but fails to reduce or refrain from alcohol use.
·The patient spends a lot of time on obtaining, using, or recovering from alcohol.
·Cravings
·The patient is unable to fulfil the patient's responsibility in offices, schools, and homes due to repeated alcohol use.
·The patient continues to use alcohol even though the alcohol use has caused and aggravated social or interpersonal problems.
·The patient abandons or reduces social, professional, or recreational activities for the sake of alcohol private use.
·The patient repeats the alcohol use even though the alcohol use is risking the health of the patient.
·The patient continues to use alcohol even though the patient is aware of the aggregation of physical or mental problem.
·Tolerance
·Withdrawal syndrome
·Craving for the use of the substance

The term "alcoholism" as used herein refers to a state in which a subject has become mentally or physically addictive to alcohol as the result of excessive alcohol intake, thereby causing a mental or physical disorder in the subject. A method of diagnosing alcoholism is, for example, International Statistical Classification of Diseases and Related Health Problems-10 (ICD-10). According to the ICD-10, when a patient has experienced 3 or more out of 6 items below at the same time for 1 month or more, or repeatedly, within the last 1 year.
·Extreme craving for drinking
·Loss of drinking control
·Withdrawal syndrome
·Evidence of tolerance
·Drinking-centered life
·Spending a lot of time on drinking activities
·Drinking even with problems

The term "mental addiction" as used herein refers to, for example, such a state as described below: having a strong desire for drinking; losing drinking control to fail to reduce alcohol consumption; spending the most of time in a day for drinking and recovery from drinking and thus ignoring any recreations other than drinking; or not abstaining from alcohol even though mental and physical problems are aggravating. The term "physical addiction" refers to, for example, such a state as described below: withdrawal symptoms such as finger twitching and sweating appear during alcohol detoxification; or requiring a larger amount of alcohol to get drunk than before.

Investigations on how to treat the alcohol use disorder have focused intensively on a disorder due to chronic alcohol intake, and it has been said that continuous alcohol abstinence is the most stable and ultimate goal for the treatment of the alcohol use disorder. However, the inventors of the present invention have found for the first time that, as in the case of chronic alcohol intake, the brain dysfunction occurs even after chronic alcohol intake and alcohol abstinence, reducing the expression amount of the oligodendrocyte marker, that is, causing the myelin sheath abnormality and the oligodendrocyte differentiation abnormality. The inventors have completed the present invention. The term "chronic" in the term "chronic alcohol intake" as used herein is not particularly limited as long as its period is long, and means, for example, a period ranging from several months to several years.

Oligodendrocytes, which are cells for forming myelin sheaths that cover axons, enhance conduction speeds of the axons through saltatory conduction. Accordingly, the myelin sheath formation with oligodendrocytes is related to higher brain functions, such as social interaction and emotional behavior. Moreover, failure of the differentiation from oligodendrocyte progenitor cells to mature oligodendrocytes results in the failure of the myelin sheath formation, leading to the brain dysfunction. According to the therapeutic agent of the present invention, the differentiation from oligodendrocyte progenitor cells to mature oligodendrocytes is enhanced, and thus the brain dysfunction and mental disorder due to the myelin sheath abnormality can be mitigated. The term "oligodendrocytes" as used herein includes oligodendrocyte progenitor cells and mature oligodendrocytes.

The term "alcoholic brain dysfunction and/or myelin sheath abnormality" as used herein refers to such a state that brain dysfunction and/or myelin sheath abnormality has occurred due to alcohol. The term "brain dysfunction" as used herein refers to such a state that a brain is damaged to show symptoms, such as cognitive and memory disorders, and anxiety disorder. The term "myelin sheath abnormality" as used herein refers to, for example, such a symptom that an abnormality appears in myelin sheath maturation or formation, or in reformation of myelin sheaths after damaged, or such a symptom that myelin sheaths are damaged or destroyed to become unable to perform appropriate information transmission. Examples of the myelin sheath abnormality include failure of the differentiation from oligodendrocyte progenitor cells to mature oligodendrocytes, and failure of myelin sheath formation due to cell number reduction of the oligodendrocyte progenitor cells and/or the mature oligodendrocytes.

The therapeutic agent of the present invention may be used in combination with an alcohol deterrent, such as cyanamide or disulfiram, an alcohol abstinence supporting drug such as acamprosate, an alcohol consumption reducing agent such as nalmefene, an anti-schizophrenia drug, an anti-depression drug, an anti-anxiety drug, an antipsychotic drug, a hypnotic drug, an anti-dementia drug, or the like. The timing of administration of the therapeutic agent of the present invention is not particularly limited, but the therapeutic agent is preferably administered when the brain dysfunction and/or myelin sheath abnormality with a mental disorder has occurred. In the case where the patient is a patient with an alcohol use disorder, the timing of the administration of the therapeutic agent of the present invention is not particularly limited and the therapeutic agent may be administered during alcohol intake or after alcohol abstinence. However, the therapeutic agent of the present invention is preferably administered when the alcoholic brain dysfunction and/or the myelin sheath abnormality has occurred during the alcohol intake or after the alcohol abstinence. Determination as to whether or not the alcoholic brain dysfunction and/or the myelin sheath abnormality has occurred may be conducted by, for example, examining the brain of the patient during the alcohol intake or after the alcohol abstinence by CT or MRI.

The term "schizophrenia" as used herein refers to a pathological condition in which the ability to gather thought, behavior, and emotions along one purpose, that is, the ability of integration is reduced over a long period of time, and a certain kind of visual hallucinations, delusions, and the like are seen through the reduction in ability of integration. A method of diagnosing schizophrenia is, for example, DSM-5 or ICD-10.

The dosage of the therapeutic agent of the present invention may be appropriately set in accordance with, for example, the age, gender, body weight, and symptoms of the subject of the administration, and the number of times, frequency, and timing of the administration. For example, in the case where the therapeutic agent is orally administered to an adult, a daily dose may be from 0.001 mg/kg to 0.5 mg/kg, preferably from 0.01 mg/kg to 0.1 mg/kg. The therapeutic agent of the present invention is not particularly limited as to its route of administration and may be administered orally or parenterally, but is preferably administered orally. Examples of a formulation of the therapeutic agent suitable for the oral administration include tablets, granules, fine granules, powders, syrups, solutions, capsules, and suspensions. Additives for formulations including: water; saccharides, such as sucrose, sorbit, and fructose; glycols, such as polyethylene glycol and propylene glycol; oils, such as sesame oil, olive oil, and soybean oil; or preservatives such as p-hydroxybenzoic acid esters may be used in the production of liquid formulations for oral administration. In addition, additives for formulations including: excipients, such as lactose, glucose, sucrose, and mannite; disintegrators, such as starch and sodium alginate; lubricants, such as magnesium stearate and talc; binders, such as methyl cellulose, polyvinyl alcohol, hydroxypropyl cellulose, and gelatin; surfactants such as fatty acid esters; or plasticizers such as glycerin may be used in the production of solid formulations, such as capsules, tablets, powders, or granules.

Moreover, as required, the therapeutic agent of the present invention may be enclosed in microcapsules (microcapsules made of, for example, hydroxymethyl cellulose, gelatin, and poly[methyl methacrylate]), or may be turned into colloid drug delivery systems (e.g., liposomes, albumin microspheres, microemulsions, nanoparticles, and nanocapsules) (see, for example, "Remington's Pharmaceutical Science 16th edition", Oslo Ed. (1980)). Further, a method of preparing a drug as a sustained release drug has been publicly-known and may be applied to the therapeutic agent of the present invention.

The present invention covers a screening method for a therapeutic agent for a mental disorder, including screening for an oligodendrocyte differentiation enhancing substance. The term "oligodendrocytes differentiation enhancing substance" as used herein refers to a substance that enhances the differentiation from neural stem cells to oligodendrocyte progenitor cells, or a substance that enhances the differentiation from neural stem cells or oligodendrocyte progenitor cells to mature oligodendrocytes.

It is preferred that the screening method of the present invention further include a step of measuring an expression amount of an oligodendrocyte marker. The term "oligodendrocyte marker" as used herein may refer to an oligodendrocyte progenitor cell marker for indicating the differentiation from neural stem cells to oligodendrocyte progenitor cells, or a mature oligodendrocyte marker or a myelin sheath marker for indicating the differentiation from neural stem cells or oligodendrocyte progenitor cells to mature oligodendrocytes. Examples thereof include: myelin sheath markers, such as myelin-associated glycoprotein (MAG), 2',3'-cyclic nucleotide 3'-phosphodiesterase (CNPase), and myelin basic protein (MBP); mature oligodendrocyte markers, such as CC1 (adenomatous polyposis coli; APC) and oligodendrocyte transcription factor 2 (Olig2); and oligodendrocyte precursor cell markers, such as platelet-derived growth factor receptor α (PDGFRα), neuron-glial antigen 2 (NG2), and Olig2. In particular, MAG, CNPase, CC1, Olig2, or PDGFRα is preferred. Olig2 is a common marker for oligodendrocyte progenitor cells and mature oligodendrocytes. The cell number of oligodendrocytes may be measured by using, as an indicator, an expression amount of an oligodendrocyte marker used in the screening method of the present invention. In the screening method of the present invention, a step of measuring the expression amount of the oligodendrocyte marker or the cell number of oligodendrocytes may include performing measurement by, for example, an immunological method, such as western blotting with an antibody, immunohistochemical staining, ELISA, or flow cytometry, mass spectrometry, or amino acid sequence analysis.

It is preferred that the screening method of the present invention further include a step of collecting a test sample from a subject to which a candidate substance has been administered, or a step of incubating oligodendrocytes with a candidate substance added thereto. Examples of the "subject" as used herein include a mouse, a rabbit, a cat, a dog, a horse, a bovine, and a simian such as a monkey. The "test sample" as used herein may be a specimen itself collected from a subject or a human subject, or a sample prepared from such specimen, and examples thereof include a brain tissue, such as a prefrontal cortex or a hippocampus, or a section thereof, an exudate, such as urine, feces, or pus, sputum, a lymph node, a tonsil, skin, lymph fluid, blood, and mucosa. In the screening method of the present invention, the subject may be appropriately changed depending on the therapeutic agent for which the screening is performed. For example, when the screening is performed for a therapeutic agent for an alcohol use disorder, the subject may be a subject administered EtOH. The wording "incubation of oligodendrocytes with a candidate substance added thereto" as used herein preferably refers to incubation of oligodendrocytes in a culture liquid with EtOH treatment.

Specifically, the screening method of the present invention preferably includes the following steps:
(1) collecting a test sample from a subject to which a candidate substance has been administered;
(2) measuring, in the test sample, an expression amount of each of one or a plurality of kinds of oligodendrocyte markers selected from MAG, CNPase, CC1, Olig2, MBP, PDGFRα, and NG2; and
(3) selecting the candidate substance as the oligodendrocyte differentiation enhancing substance when the expression amount of each of the one or the plurality of oligodendrocyte markers is enhanced in the subject with the candidate substance administered thereto as compared to a subject without the candidate substance.

Specifically, the step (3) is such that the subject with the candidate substance administered thereto is compared to the subject without the candidate substance as to the expression amount of each of the one or the plurality of oligodendrocyte markers, and when the expression amount of each of the one or the plurality of oligodendrocyte markers is more enhanced in the subject with the candidate substance administered thereto as compared to the subject without the candidate substance, the candidate substance is selected as the oligodendrocyte differentiation enhancing substance. The expression amount of each of the one or the plurality of oligodendrocyte markers in the subject without the candidate substance may be measured through the steps (1) and (2).

Through the steps (1) to (3), an oligodendrocyte differentiation enhancing substance can be selected, thereby making it possible to screen for a therapeutic agent for a mental disorder of the present invention. In the screening method of the present invention, the "candidate substance" is not particularly limited and may be any substance that is estimated as being possibly effective against the mental disorder, but examples thereof include a protein, a peptide, a non-peptide compound, a synthetic low-molecular compound, a natural compound, a cell extraction, a cell culture supernatant, a microorganism fermentation product, an extract derived from marine organism, and a plant extract.

The present invention also covers a method of examining prognosis of a mental disorder, including measuring, in a test sample collected from a human subject, one or a plurality of kinds selected from oligodendrocyte markers, oligodendrocyte differentiation modulators, oligodendrocyte differentiation regulators, and oligodendrocyte-derived products. According to the examination method of the present invention, the prognosis of the mental disorder can be estimated, and thus an appropriate treatment method can be selected. For example, when a value of each of one or a plurality of kinds selected from oligodendrocyte markers, oligodendrocyte differentiation modulators, oligodendrocyte differentiation regulators, and oligodendrocyte-derived products in a human subject is equal to or less than a preset reference value of each of the one or the plurality of kinds selected from the oligodendrocyte markers, the oligodendrocyte differentiation modulators, the oligodendrocyte differentiation regulators, and the oligodendrocyte-derived products, it can be estimated that the human subject has not been recovered from the mental disorder. In this way, when the human subject is a patient with a mental disorder and is still under treatment for the mental disorder, the treatment for the mental disorder can be continuously performed, and when the human subject is not under treatment for the mental disorder, the start of the treatment for the mental disorder can be proposed. Moreover, for example, when the value is equal to or more than the reference value, it can be estimated that the human subject has been recovered from the mental disorder by the treatment. The term "recover" as used herein means that the brain function is normal.

The term "oligodendrocyte differentiation modulator" as used herein refers to a modulator involved in the differentiation from neural stem cells to oligodendrocyte progenitor cells, or the differentiation from the neural stem cells or the oligodendrocyte progenitor cells to mature oligodendrocytes. Examples thereof include triiodothyronine (T3), thyroxine (T4), platelet-derived growth factor (PDGF), basic fibroblast growth factor (bFGF), neurotrophin-3 (NT3), insulin-like growth factors (IGF), sonic hedgehog (SHH), leukemia inhibitory factor (LIF), and myelin regulatory factor (MYRF). In particular, T3 or T4 is preferred. The term "oligodendrocyte differentiation regulator" as used herein refers to a micro-RNA (miRNA) for regulating the differentiation from the neural stem cells to oligodendrocyte progenitor cells, or the differentiation from the neural stem cells or the oligodendrocyte progenitor cells to the mature oligodendrocytes. In recent years, the use of a miRNA as a biomarker has been more widely adopted. Regarding the differentiation of oligodendrocytes, it has been found that a specific miRNA is involved in the differentiation as a differentiation regulator (see, for example, Dylan A et al., Front Cell Dev Biol., 2016 Jun 17; 4: 59, John-Mark K et al., The International Journal of Biochemistry & Cell Biology, 2015 65; 134: 138). The "miRNA for regulating the differentiation of oligodendrocytes" as used herein is, for example, a miRNA whose expression is increased in the differentiation process of oligodendrocytes, and specific examples thereof include miR-219, miR-388, miR-138, and miR-9. The "oligodendrocyte-derived product" as used herein is not particularly limited as long as the product is a lipid for forming myelin sheath, but examples thereof include cerebroside and sulfatide.

In the examination method of the present invention, a method of measuring one or a plurality of kinds selected from oligodendrocyte markers, oligodendrocyte differentiation modulators, oligodendrocyte differentiation regulators, and oligodendrocyte-derived products is not particularly limited as long as the method can determine one or a plurality of kinds selected from oligodendrocyte markers, oligodendrocyte differentiation modulators, oligodendrocyte differentiation regulators, and oligodendrocyte-derived products in the test sample. The measurement of the oligodendrocyte marker or the oligodendrocyte differentiation modulator may be performed at a protein level or at an RNA level. Detection or quantification at the protein level may be performed by an immunological method or the like. Moreover, detection or quantification at the RNA level may be performed by, for example, RT-PCR, Northern blotting, or in-situ hybridization. Moreover, for measuring the oligodendrocyte differentiation regulator, the measurement may be performed by, for example, extracting the miRNA in exosomes in the test sample collected from the human subject. The exosomes may be obtained from the test sample by a method known per se. Examples thereof include a centrifugal method, a density gradient centrifugal method, a super-centrifugal method, a polymer precipitation method, a separation method utilizing size exclusion chromatography, and a separation method utilizing affinity. The extraction of the miRNA from the exosomes is carried out by a method known per se. For example, an RNA may be extracted with an RNA extraction agent or the like, and a miRNA may be then extracted therefrom with a miRNA extraction kit or the like. For measuring the oligodendrocyte-derived product, the measurement may be performed by, for example, gas chromatography (GC/MS) or liquid chromatography (LC/MS). In the examination method of the present invention, examples of the "test sample" include a brain tissue, such as a prefrontal cortex or a hippocampus, or a section thereof, and a body fluid, such as blood, urine, lymph fluid, or saliva, collected from a human subject.

### Examples

To help understanding of the present invention, the present invention is specifically described below by way of Examples, but it goes without saying that the present invention is not limited thereto. In Examples, the wording "immediately after alcohol intake" in the drawings means 4 days later from the last alcohol intake, and the wording "after alcohol intake and alcohol abstinence" means another 3 weeks later from the time immediately after alcohol intake. Moreover, in Examples, mice with alcohol intake mimic a state with chronic alcohol intake.

### (Example 1) Behavioral Experiments on Mice immediately after Alcohol Intake

In this Example, behaviors of mice immediately after alcohol intake were evaluated.

Male C57BL/6J mice at the age of 4 weeks (P28) were used. The mice were accommodated in a plastic cage and maintained at a light-dark cycle of 12 hours (lighting at 8:00 A.M.) with ad libitum access to feed and water.

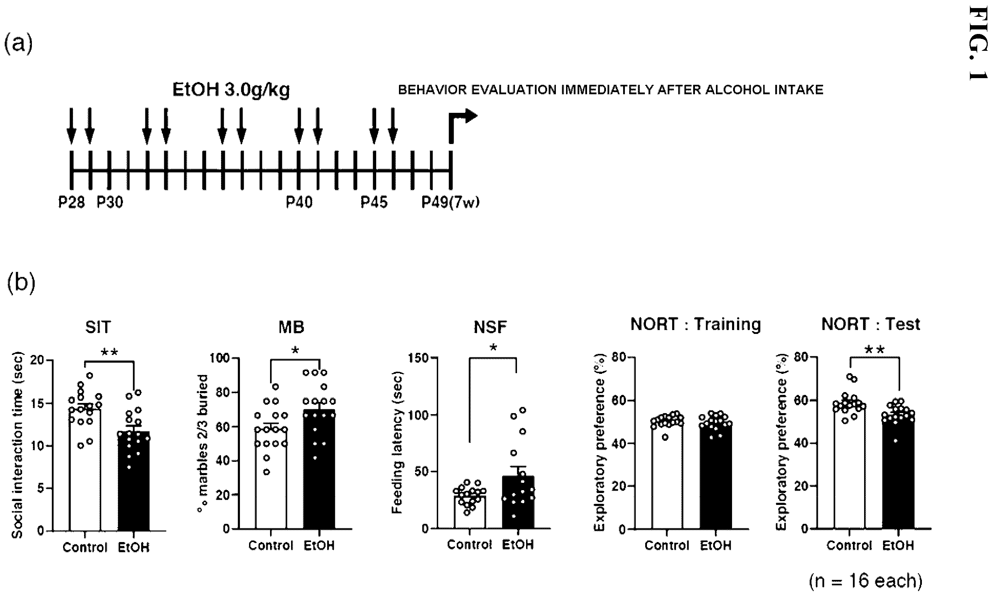

Sixteen mice were orally administered EtOH (3.0 g/kg; diluted to 25% with sterile water) intermittently from P28 to P45 for 10 days in total (once a day) according to the schedule of FIG. 1(a) . Sixteen control mice were orally administered sterile water in the same amount as that in the mice administered EtOH.

All the behavioral experiments were performed between 10:00 A.M. and 6:00 P.M. To obtain an indicator of sociality, a social interaction test was conducted. To obtain an indicator of anxiety behavior, a marble-burying behavior test and a novelty suppressed feeding test were conducted. To obtain an indicator of cognitive function, a novel object recognition test was conducted. To reduce influences from previous experiments, the social interaction test, the marble-burying behavior test, the novelty suppressed feeding test, and the novel object recognition test were conducted in the stated order. Before the behavioral experiments were conducted, the test mice were acclimated in a laboratory for 2 hours or more.

### (Social Interaction Test (SIT))

The social interaction test was conducted by a method in conformity with a previous report (Mouri A et al., J Neurosci. 2012 Mar 28; 32(13): 4562-80). The social interaction test was conducted in an open box (30 cm×30 cm×35 cm), which was square, made of gray non-reflective acrylic, and opened on the top, under illumination of 50 lux. The test mice had been acclimated by being solely placed in the open box for 10 minutes daily for 2 days before the social interaction test. On the day of the test, one of the mice was placed in the open box for 10 minutes together with another C57BL/6J mouse, which was of the same age and the same gender as the test mouse and was randomly selected as a new partner from a home cage other than a home cage of the test mouse. A time length of a social interaction (e.g., smelling each other, grooming each other, pursuing, mounting, or crawling) was recorded. A passive interaction (body touching as the result of sitting or lying) was not included in the time length of the social interaction.

### (Marble-Burying Behavior Test (MB))

The marble-burying behavior test was conducted by a method in conformity with a previous report (Alkam T et al., Behav Brain Res. 2013 Feb 15; 239: 80-9). The marble-burying behavior test was conducted in a plastic cage (26 cm×21 cm×15 cm) with a floor material (sawdust of 3 cm in depth) laid on its bottom under illumination of 40 lux. On the sawdust inside the cage, 12 marbles were placed with constant intervals therebetween. A test mouse was placed at a corner of the cage with the marbles and let freely search inside the cage for 15 minutes. When 2/3 of the surface area of a marble was covered with the sawdust, the marble was counted as being buried.

### (Novelty Suppressed Feeding Test (NSF))

The novelty suppressed feeding test was conducted by a method in conformity with a previous report (Lu Q et al., Behav Brain Res. 2019 Oct 17; 372: 112053). The test mice were subjected to fasting with ad libitum access to water from 36 hours before the test. The novelty suppressed feeding test was conducted in a plastic cage (26 cm×21 cm×15 cm) with the floor material (sawdust of 2 cm in depth) laid on its bottom under illumination of 300 lux. At the center of the cage, a 10 cm petri dish with circular white filter paper (diameter; 10 cm) therein was placed, and a grain of mouse feed was placed on the petri dish. A test mouse was placed at a corner of the cage and let freely search inside the cage for 5 minutes. A latency time taken for feed intake was recorded.

### (Novel Object Recognition Test (NORT))

The novel object recognition test was conducted by a method in conformity with a previous report (Mouri A et al., J Neurosci. 2012 Mar 28; 32(13): 4562-80). A test procedure of the novel object recognition test includes three sessions, namely habituation, training, and retention. The test mice were acclimated by being placed in a square open box (30 cm×30 cm×35 cm) for 10 minutes daily for 3 days (Habituation). In the training session, two objects (out of a golf ball, a wooden circular column, and a square pyramid, which were different in shape and color but similar in size) were placed inside the box, and a test mouse was placed in a middle portion of the box. How long the mouse was exploring the two objects was recorded for 10 minutes. A time period for which the mouse turned its head toward any one of the objects, touched any one of the objects, and smelled any one of the objects was considered as object exploration and measured (Training). In the test session (Test), 24 hours later from the training session, the test mouse was returned to the same box, in which one of the objects used in the training was replaced with a novel object. After that, the test mouse was let freely explore for 10 minutes, and a time period for which the mouse explored each object was measured. As an indicator of the recognition function, a ratio of the time taken for exploring any one of the two objects or the novel object to the total time taken for exploring the two objects was used.

The results are shown in FIG. 1(b). In the social interaction test, the alcohol intake group (EtOH) showed a significant reduction in contact time with the partner mouse. Moreover, in the marble-burying behavior test and the novelty suppressed feeding test, the alcohol intake caused an increase in number of buried marbles and an increase in latency to feed. Further, in the novel object recognition test, a significant reduction in approach time to the novel object was found. The results demonstrated that, immediately after the alcohol intake, the reduction in social interaction, the anxiety-like behavior, and the recognition dysfunction were induced.

### (Example 2) Behavioral Experiments on Mice after Alcohol Intake and Alcohol Abstinence

In this Example, behaviors of mice after alcohol intake and alcohol abstinence were evaluated.

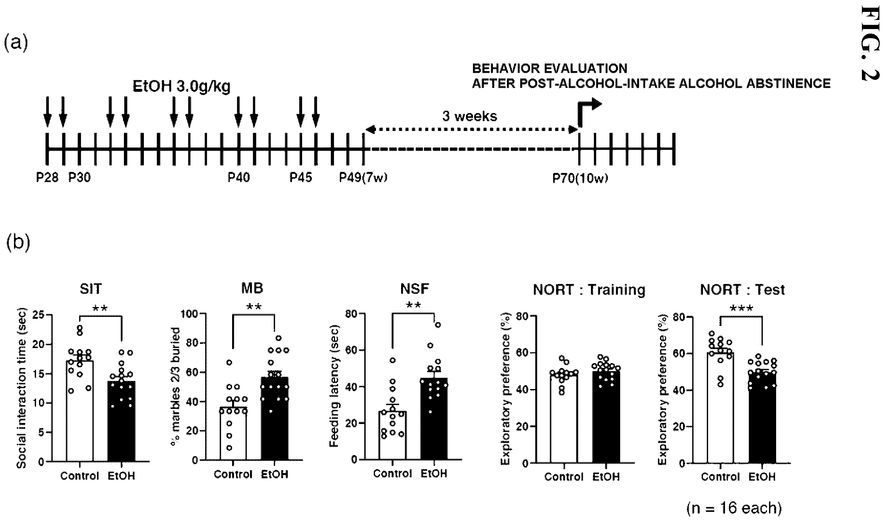

Sixteen mice administered EtOH, which had been prepared in the same manner as in Example 1, were subjected to alcohol abstinence for 3 weeks. After that, the behaviors of the mice were evaluated (FIG. 2(a)). Sixteen control mice were orally administered sterile water in the same amount as that in the mice administered EtOH. The behavioral experiments were conducted in the same manner as in Example 1.

The results are shown in FIG. 2(b). In the social interaction test, the alcohol intake group (EtOH) showed a significant reduction in contact time with the partner mouse. Moreover, in the marble-burying behavior test and the novelty suppressed feeding test, it was found that the alcohol intake caused an increase in number of buried marbles and an increase in latency to feed. Further, in the novel object recognition test, a significantly reduction in approach time to the novel object was found. The results demonstrated that, even after the alcohol intake and alcohol abstinence, the behavioral disorders, such as the reduction in social interaction, the anxiety-like behavior, and the recognition dysfunction, were protracted for a long time.

### (Example 3) Determination of Myelin Sheath Abnormalities in Mice immediately after Alcohol Intake and after Alcohol Intake and Alcohol Abstinence

In this Example, myelin sheath abnormalities in mice immediately after alcohol intake, and after alcohol intake and alcohol abstinence were determined with a myelin sheath marker (MAG, CNPpase) and compared to each other. A myelin sheath amount was analyzed by western blotting.

Mice (8 mice for each group) were administered EtOH in the same manner as in Examples 1 and 2. Control mice (8 mice for each group) were orally administered sterile water in the same amount as that in the mice administered EtOH. The western blotting was conducted by a method in conformity with a previous report (Kunisawa K et al., Eur J Pharmacol. 2017 Feb 5; 796: 122-130). A prefrontal cortex and a hippocampus were collected from each mouse and frozen at -80°C to be used for the western blotting. Each of the frozen prefrontal cortex and hippocampus was homogenized in an ice-cooled homogenization buffer by ultrasonic treatment. Samples thus prepared were centrifuged at 13,000 rpm at 4°C for 15 minutes to provide supernatants, and protein concentrations in the supernatants were measured with a Quick StartTM Bradford 1x dye reagent (Cat# 5000205, Bio-Rad, Berkeley, CA, USA), and standardized to 2.0 µg/µL. Each protein sample thus prepared was subjected to electrophoresis with 10% (w/v) SDS-PAGE and subsequently transferred to a 0.22 mm-thick PVDF membrane (Cat# GVWP04700, Millipore, Billerica, MA, USA). The membrane was subjected to blocking with 5% skim milk in TBST for 60 minutes at room temperature and caused to react with one of primary antibodies at 4°C overnight. Then, the PVDF membrane was washed with TBST (3 times, 10 minutes), and caused to react with an appropriate HRP-labeled secondary antibody at room temperature for 2 hours. After that, the PVDF membrane was washed again with TBST (3 times, 10 minutes), and caused to react with an Immobilon Forte Western HRP substrate (Cat# WBLUF0100, Millipore). Immunoreactive bands were visualized with ATTO LuminoGraphI (Cat# WSE-6100, ATTO, Tokyo, Japan). The primary antibodies used were rabbit anti-MAG (1:1,000, Cat# 9043, Cell Signaling Technology, Danvers, MA, USA), rabbit anti-CNPase (1:1,000, Cat# 5664, Cell Signaling Technology), and mouse anti-β-actin (1:1,000, Cat# A5441, Sigma-Aldrich).

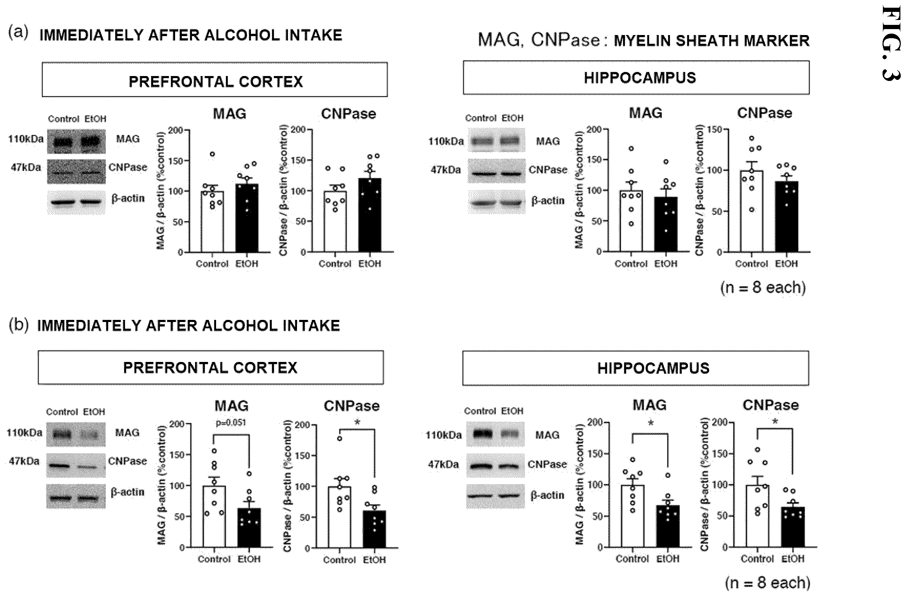

The results are shown in FIGS. 3. The prefrontal cortexes and hippocampuses of the mice immediately after the alcohol intake showed no significant changes in myelin sheath amount, (FIG. 3(a)). However, the prefrontal cortexes and hippocampuses of the mice after the alcohol intake and alcohol abstinence showed significant reductions in myelin sheath amount (FIG. 3(b)). The results suggest a possibility that the myelin sheath abnormality is involved in the behavioral abnormalities to be found after the alcohol intake and alcohol abstinence.

### (Example 4) Determination of Cell Numbers of Oligodendrocytes in Mice Immediately After Alcohol Intake and After Alcohol Intake and Alcohol Abstinence

In this Example, the cell numbers of oligodendrocyte progenitor cells and mature oligodendrocytes in prefrontal cortexes and hippocampuses of the mice immediately after alcohol intake and after alcohol intake and alcohol abstinence were determined with an oligodendrocyte progenitor cell marker (PDGFRα) and a mature oligodendrocyte marker (CC1) and compared to each other. The cell numbers of oligodendrocytes were analyzed by immunohistochemical staining.

Mice (4 mice for each group) were administered EtOH in the same manner as in Examples 1 and 2. Control mice (4 mice for each group) were orally administered sterile water in the same amount as that in the mice administered EtOH. The immunohistochemical staining was conducted by a method in conformity with a previous report (Kunisawa K et al., J Neurochem. 2018 Nov; 147 (3) : 395-408) . Frozen slices of prefrontal cortexes and hippocampuses of the mice prepared were heated in a 10 mM citrate buffer solution (pH 6.0) with a microwave oven for 5 minutes. After washed with PBS, the slices were subjected to blocking with 5% normal horse serum (Cat# S-2000, Vector Laboratories, Inc., Burlingame, CA, USA) in PBST for 1 hour. After that, the slices were caused to react with one of primary antibodies at 4°C overnight. After washed with PBST, the slices were caused to react with a secondary antibody (1:2,000) at room temperature for 3 hours. The primary antibodies used were a mouse anti-CC1 antibody (1:500; Cat# MABF850, Millipore) and a goat anti-PDGFRα antibody (1:200; Cat# AF1062, R&D Systems Inc.)

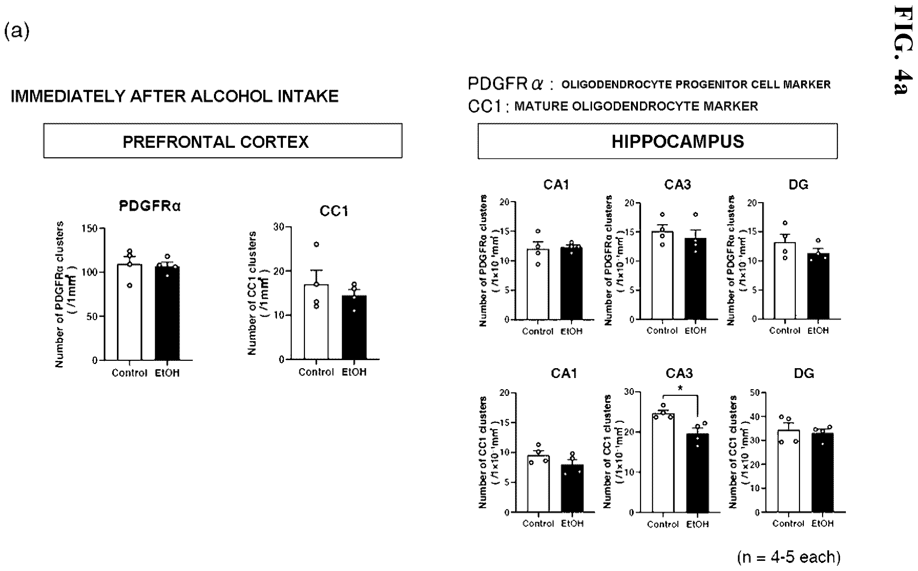

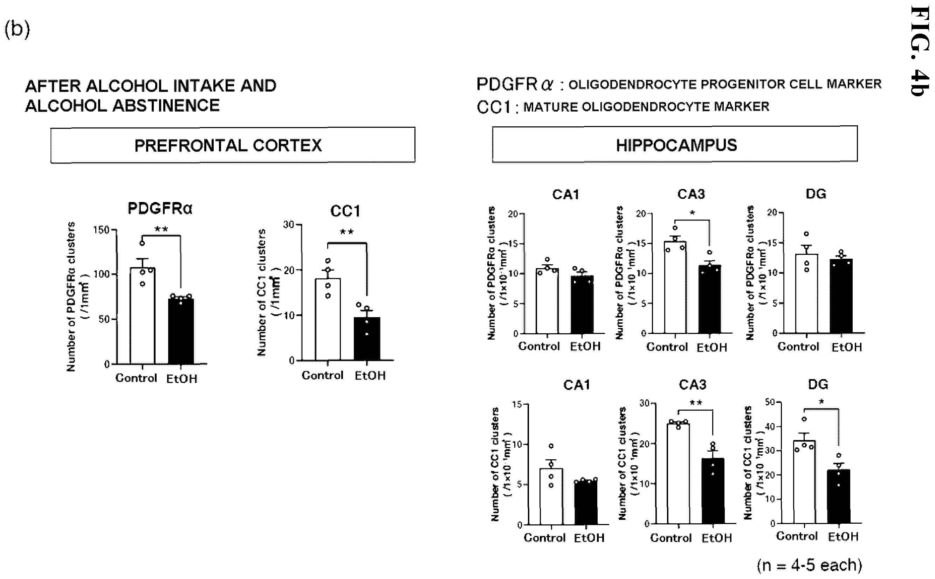

The results are shown in FIGS. 4. The prefrontal cortexes and hippocampuses of the mice immediately after the alcohol intake showed no significant changes in cell numbers of oligodendrocytes (FIG. 4(a)). However, the prefrontal cortexes and hippocampuses of the mice after the alcohol intake and alcohol abstinence showed significant reductions in cell numbers of oligodendrocyte progenitor cells and mature oligodendrocytes (FIG. 4(b)), as in the case of the myelin sheath abnormality. The results suggest a possibility that the myelin sheath abnormality to be found after alcohol intake and alcohol abstinence is caused by the reduction in cell number of oligodendrocytes.

### (Example 5) Effect 1 of Clemastine on Mice after Alcohol Intake and Alcohol Abstinence

In this Example, clemastine was administered to mice after alcohol intake and alcohol abstinence, and behaviors of the mice were evaluated.

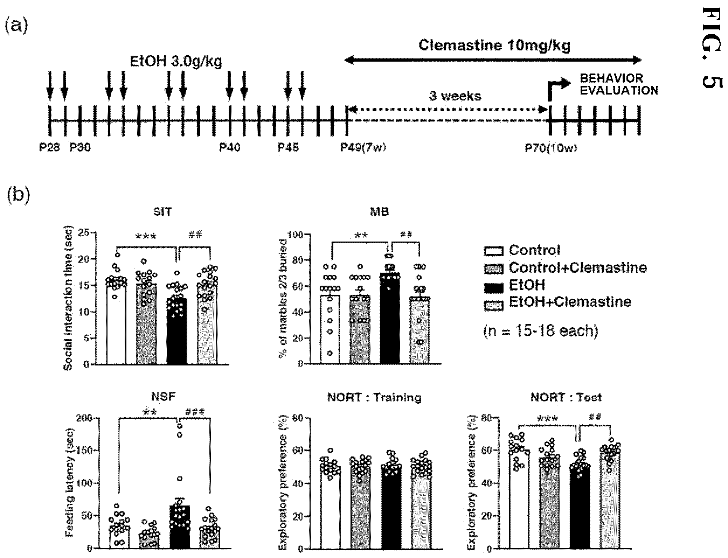

Mice were administered EtOH in the same manner as in Example 2 (FIG. 5(a)). Control mice were orally administered sterile water in the same amount as that in the mice administered EtOH. Clemastine (Cat# 14976-53-7, R&D Systems Inc., Minneapolis, MN, USA) was suspended in sterile water. The suspension was orally administered as clemastine (10 mg/kg) to the mice every day from the end of the administration of EtOH to the final day of the behavioral tests (FIG. 5(a)). After that, behaviors of a control group (n=18), a control+clemastine group (n=15), an EtOH group (n=17), and an EtOH+clemastine group (n=18) were evaluated in the same manner as in Example 1.

The results are shown in FIG. 5(b). In the social interaction test, the marble-burying behavior test, the novelty suppressed feeding test, and the novel object recognition test, the clemastine administration significantly remitted the social interaction reduction, the anxiety-like behavior, and the recognition dysfunction to be found after the alcohol intake and alcohol abstinence. The results suggest a possibility that clemastine having a enhancing effect on myelin sheath formation may serve as a novel therapeutic agent for an alcohol use disorder.

### (Example 6) Determination of Abnormality in Differential Proliferation of Oligodendrocytes in Mice after Alcohol Intake and Alcohol Abstinence

In this Example, a percentage of differential proliferation of oligodendrocytes in hippocampuses of mice after alcohol intake and alcohol abstinence was measured.

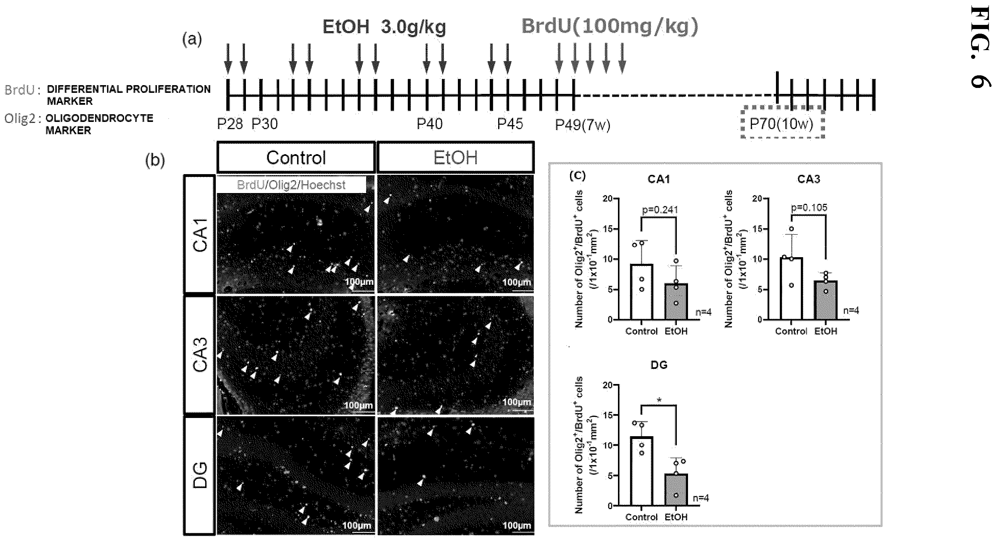

Mice (4 mice) were administered EtOH in the same manner as in Example 4 (FIG. 6(a)), and immunohistochemical staining was conducted on the hippocampuses of the mice after alcohol abstinence. Control mice (4 mice) were orally administered sterile water in the same amount as that in the mice administered EtOH. For the differential proliferation of oligodendrocytes, bromodeoxyuridine (Brdu) was used as an indicator of a differential proliferation marker. After the end of the EtOH intake, BrdU (100 mg/kg) was intraperitoneally administered to the mice once every day for 5 days (FIG. 6(a)). After alcohol abstinence for about 3 weeks, the cell numbers of oligodendrocytes co-positive for an oligodendrocyte marker (Olig2) and a differential proliferation marker (BrdU) were determined by the immunohistochemical staining and compared to each other, in order to investigate whether or not the percentage of differential proliferation of oligodendrocytes had been reduced. The primary antibodies used were a mouse anti-Olig2 antibody (Cat# MABN50, Merck) and a rat anti-BrdU antibody (Cat# ab6326, abcam).

The results are shown in FIG. 6(b) and FIG. 6(c). FIG. 6(b) shows the results of the immunohistochemical staining of the hippocampuses. In FIG. 6(b), the symbols "Δ" indicate the cell numbers of oligodendrocytes co-positive for the oligodendrocyte marker (Olig2) and the differential proliferation marker (BrdU). FIG. 6(c) shows the cell numbers of oligodendrocytes co-positive for Olig2 and BrdU. In the dentate gyrus (DG) after the alcohol intake and alcohol abstinence, the differential proliferation of oligodendrocytes was significantly reduced. The results suggest a possibility that the reduction in cell number of the oligodendrocytes to be found after the alcohol intake and alcohol abstinence is caused by the abnormality in the differential proliferation.

### (Example 7) Investigation of Alcohol Vulnerability After Alcohol Intake and Alcohol Abstinence in Genetically Modified Animal with Inhibited Myelin Sheath Formation.

In this Example, alcohol vulnerability after alcohol intake and alcohol abstinence in a genetically modified animal with inhibited myelin sheath formation was investigated.

### (Preparation of Genetically Modified Animal with Inhibited Myelin Sheath Formation)

A mouse with oligodendrocyte progenitor cells from which a myelin regulatory factor (myrf) was deleted (hereinafter referred to as "NG2-CreERT;Myrf-Flox mouse") was prepared. An NG2-CreERT genetically modified mouse, which has incorporated therein a tamoxifen-induced Cre recombinase on an NG2 gene promoter serving as an oligodendrocyte progenitor cell marker, the recombinase recognizing LoxP sequences and causing recombination thereto, was crossbred with an Myrf-Flox genetically modified mouse, which has the exon region of a myelin regulatory factor (Myrf) gene sandwiched between LoxP and LoxP, the gene being known as the myelin regulatory factor, to prepare the NG2-CreERT;Myrf-Flox mouse. Administration of tamoxifen to the mouse enables the myelin sheath formation to be inhibited specifically in oligodendrocytes in a stage-specific manner.

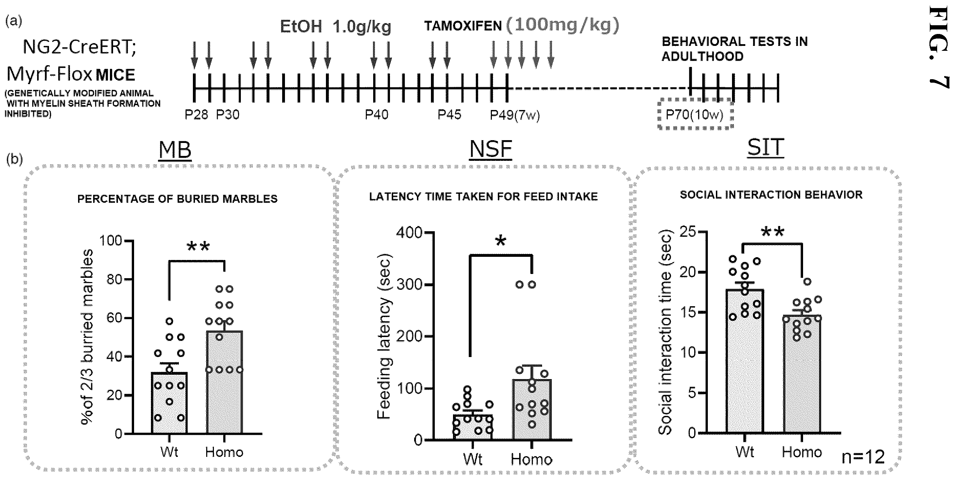

NG2-CreERT;Myrf-Flox mice or genetically unmodified mice (WT) (12 mice each) were administered EtOH, and the behaviors of the mice were evaluated after alcohol abstinence for about 3 weeks in the same manner as in Example 2 (FIG. 7(a)). Specifically, after the NG2-CreERT;Myrf-Flox mice or genetically unmodified mice (WT) (12 mice each) were administered EtOH in such a small amount (1 g/kg) as not to cause brain dysfunction in mice usually, these mice were intraperitoneally administered tamoxifen (100 mg/kg) once a day for 5 days. After alcohol abstinence for 3 weeks, various behavioral tests (the marble-burying behavior test, the novelty suppressed feeding test, and the social interaction test) were conducted. In FIGS. 7, the label "Homo" indicates NG2-CreERT;Myrf-Flox mice.

The results are shown in FIG. 7(b). In the marble-burying test (MB), the novelty suppressed feeding test (NSF), and the social interaction test (SIT), alcohol vulnerability was recognized when the oligodendrocyte differentiation and the myelin sheath formation were inhibited. This demonstrated that the inhibition of the oligodendrocyte differentiation aggravated the brain dysfunction caused by alcohol. The results suggest that the abnormality in the oligodendrocyte differentiation is involved in the behavioral abnormalities due to excessive alcohol intake.

### (Example 8) Effect 2 of Clemastine on Mice after Alcohol Intake and Alcohol Abstinence

In this Example, clemastine was administered to mice after alcohol intake and alcohol abstinence, and a myelin sheath formation enhancing effect in prefrontal cortex was investigated.

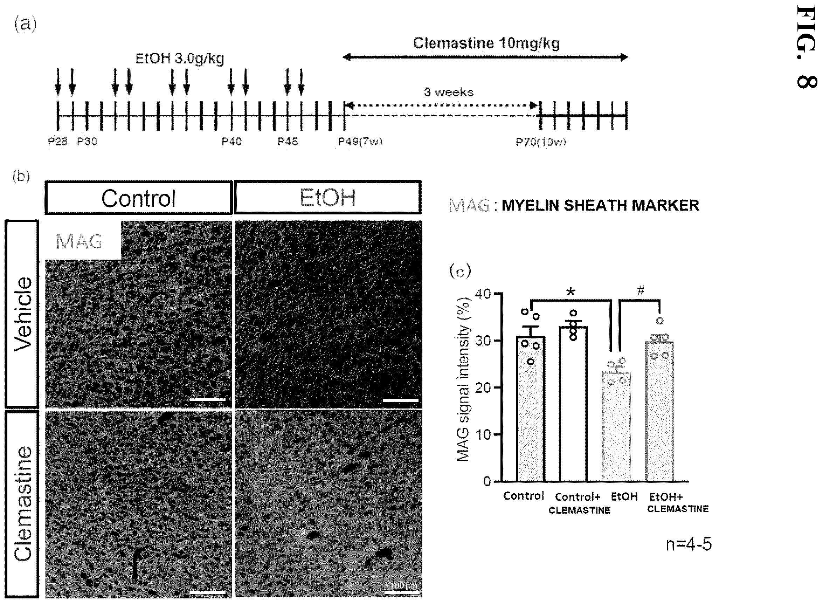

Mice were administered EtOH in the same manner as in Example 6 (FIG. 8(a)). Control mice were orally administered sterile water in the same amount as that in the mice administered EtOH. Clemastine (Cat# 14976-53-7, R&D Systems Inc., Minneapolis, MN, USA) was suspended in sterile water. The suspension was orally administered as clemastine (10 mg/kg) to the mice every day from the end of the EtOH administration to the final day of the behavioral tests. Myelin sheath formation enhancing effects in a control group (5 mice), a control+clemastine group (4 mice), an EtOH group (4 mice), and an EtOH+clemastine group (5 mice) were analyzed by immunohistochemical staining with a myelin sheath marker (MAG). The primary antibody used was a rabbit anti-MAG antibody (Cat# 9043, CST).

The results are shown in FIG. 8(b). FIG. 8(b) shows the results of the immunohistochemical staining of the prefrontal cortexes. FIG. 8(c) shows the signal intensity of the MAG measured with ImageJ. The myelin sheath abnormality to be found in the prefrontal cortexes after the alcohol intake and alcohol abstinence were significantly remitted by the clemastine administration.

### Industrial Applicability

As described in detail above, it was recognized that the cell number of oligodendrocytes was reduced and myelin sheath abnormality occurred in a mental disorder, especially an alcohol use disorder. As a result, the therapeutic agent of the present invention including clemastine or a pharmacologically acceptable salt thereof as an active ingredient is useful as a therapeutic agent for a mental disorder. Moreover, clemastine is useful because clemastine has been commercially available as an antihistamine drug, and, therefore, its safety against a human has been sufficiently proven. Further, according to the screening method of the present invention, a novel therapeutic agent for a mental disorder can be screened for.

## Claims

1. A therapeutic agent for a mental disorder, comprising clemastine or a pharmacologically acceptable salt thereof as an active ingredient.

2. The therapeutic agent according to claim 1, wherein the mental disorder is an alcohol use disorder and/or schizophrenia.

3. The therapeutic agent according to claim 2, wherein the alcohol use disorder is alcoholic brain dysfunction and/or myelin sheath abnormality.

4. A screening method for a therapeutic agent for a mental disorder, comprising screening for an oligodendrocyte differentiation enhancing substance.

5. The screening method for a therapeutic agent for a mental disorder according to claim 4, comprising a step of measuring an expression amount of an oligodendrocyte marker.

6. The screening method according to claim 4 or 5, wherein the mental disorder is an alcohol use disorder and/or schizophrenia.

7. The screening method according to claim 6, wherein the alcohol use disorder is alcoholic brain dysfunction and/or myelin sheath abnormality.

8. The screening method according to claim 4 or 5, further comprising a step of collecting a test sample from a subject, to which a candidate substance has been administered, or a step of incubating oligodendrocytes with a candidate substance added thereto.

9. The screening method according to claim 4 or 5, comprising at least the following steps (1) to (3):
(1) collecting a test sample from a subject, to which a candidate substance has been administered;
(2) measuring, in the test sample, an expression amount of each of one or a plurality of kinds of oligodendrocyte markers selected from MAG, CNPase, CC1, Olig2, MBP, PDGFRα, and NG2; and
(3) selecting the candidate substance as the oligodendrocyte differentiation enhancing substance when the expression amount of each of the one or the plurality of kinds of oligodendrocyte markers is enhanced in the subject with the candidate substance administered thereto as compared to a subject without the candidate substance.

10. A method of examining prognosis of a mental disorder, comprising measuring, in a test sample collected from a human subject, one or a plurality of kinds selected from oligodendrocyte markers, oligodendrocyte differentiation modulators, oligodendrocyte differentiation regulators, and oligodendrocyte-derived products.
